(19) Europäisches Patentamt
European Patent Office
Office européen des brevets

(11) Veröffentlichungsnummer: **0 030 650**
**A1**

(12) # EUROPÄISCHE PATENTANMELDUNG

(21) Anmeldenummer: 80107305.7

(22) Anmeldetag: 24.11.80

(51) Int. Cl.³: C 07 D 401/12
A 61 K 31/64
//(C07D401/12, 209/46, 211/96),
(C07D401/12, 217/24, 211/96)

(30) Priorität: 01.12.79 DE 2948434

(43) Veröffentlichungstag der Anmeldung:
24.06.81 Patentblatt 81/25

(84) Benannte Vertragsstaaten:
AT BE CH DE FR GB IT LI NL

(71) Anmelder: HOECHST AKTIENGESELLSCHAFT
Zentrale Patentabteilung Postfach 80 03 20
D-6230 Frankfurt/Main 80(DE)

(72) Erfinder: Hitzel, Volker, Dr.
Kantstrasse 1b
D-6238 Hofheim am Taunus(DE)

(72) Erfinder: Weyer, Rudi, Dr.
Johann-Strauss-Strasse 43
D-6233 Kelkheim (Taunus)(DE)

(72) Erfinder: Geisen, Karl, Dr.
Jahnstrasse 43
D-6000 Frankfurt am Main(DE)

(72) Erfinder: Regitz, Günter, Dr.
Dachbergstrasse 68
D-6236 Bad Soden am Taunus(DE)

(54) **1-Piperidinsulfonylharnstoffe, Verfahren zu ihrer Herstellung, pharmazeutische Präparate auf Basis dieser Verbindungen sowie ihre Verwendung.**

(57) Sulfonylharnstoffe der Formel

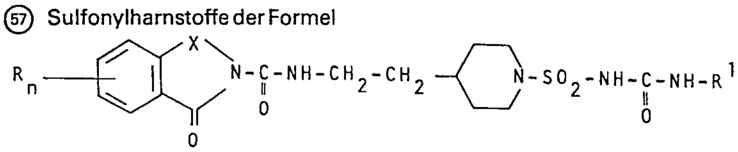

worin n, R, $R^1$ und X die angegebenen Bedeutungen haben, sowie deren physiologisch verträglichen Salze, pharmazeutische Präparate auf Basis dieser Verbindungen und ihre Verwendung bei der Behandlung des Diabetes.

EP 0 030 650 A1

Croydon Printing Company Ltd.

HOECHST AKTIENGESELLSCHAFT HOE 79/F 328 Dr.D/sch

# 1-Piperidinsulfonylharnstoffe, Verfahren zu ihrer Herstellung, pharmazeutische Präparate auf Basis dieser Verbindungen sowie ihre Verwendung

Die Erfindung betrifft Sulfonylharnstoffe der Formel

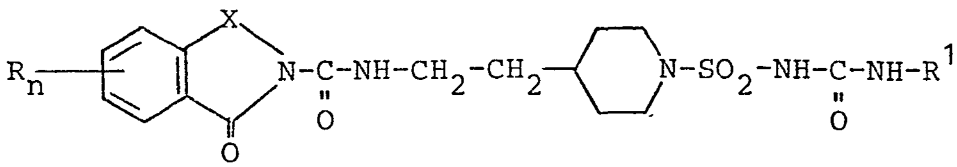

die als Substanz oder in Form ihrer physiologisch verträglichen Salze blutzuckersenkende Eigenschaften besitzen und sich durch starke und langanhaltende Senkung des Blutzuckerspiegels auszeichnen.

In der Formel bedeuten

n 1 oder 2

R Wasserstoff, Alkyl mit 1 bis 4 Kohlenstoffatomen, Alkoxy mit 1 bis 4 Kohlenstoffatomen oder Halogen, wobei falls n 2 ist, die R gleich oder verschieden sein können,

X eine $-CH_2-$, $-CH_2-CH_2-$ oder eine $-CH(CH_3)-$Gruppe

$R^1$ Alkyl von 2 bis 8 C-Atomen, Cycloalkyl, Alkylcycloalkyl, Dialkylcycloalkyl, Cycloalkylalkyl, Cycloalkenyl, Alkylcycloalkenyl mit jeweils 5 bis 9 C-Atomen, Methylcyclopentylmethyl Cyclohexenylmethyl, Chlorcyclohexyl, Methoxycyclohexyl, Bicycloheptyl, Bicycloheptenyl, Bicycloheptylmethyl, Bicycloheptenylmethyl, Bicyclooctyl, Nortricyclyl, Adamantyl oder Benzyl.

In der allgemeinen Formel bedeutet R vorzugsweise Wasserstoff, Methyl, Methoxy und Halogen, insbesondere Chlor. Besonders bevorzugt ist für R Wasserstoff. X bedeutet vorzugsweise die $CH_2$-Gruppe. $R^1$ ist vorzugsweise Butyl, Cyclohexyl, Methylcyclohexyl, besonders bevorzugt ist Cyclohexyl. Als bicyclische Reste kommen beispielsweise in Frage: Bicyclo[2.2.1]-heptyl, Bicyclo[2.2.1]heptylmethyl sowie die entsprechenden ungesättigten Reste und der Bicyclo[2.2.2]octylrest.

Die Erfindung betrifft ferner Verfahren zur Herstellung dieser Sulfonylharnstoffe, pharmazeutische Präparate, die diese enthalten oder aus ihnen bestehen sowie ihre Verwendung zur Behandlung des Diabetes.

Die Verfahren zur Herstellung sind dadurch gekennzeichnet, daß man

a) mit der Gruppe

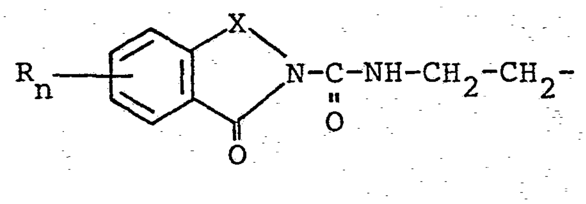

in 4-Stellung substituierte 1-Piperidinsulfonyl-isocyanate, -carbaminsäureester, -thiolcarbaminsäureester, -harnstoffe, -semicarbazide oder -semicarbazone mit einem Amin $R^1-NH_2$ oder dessen Salzen umsetzt oder Sulfonamide der Formel

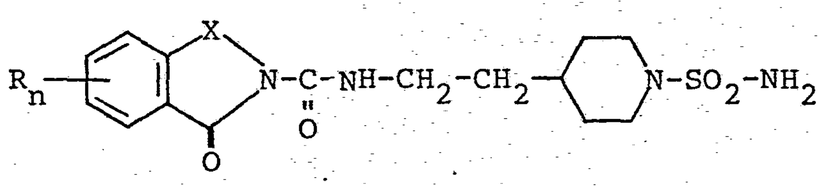

oder deren Salze mit $R^1$-substituierten Isocyanaten, Carbaminsäureestern, Thiolcarbaminsäureestern, Carbaminsäurehalogeniden oder Harnstoffen umsetzt,

b) entsprechend substituierte 1-Piperidinsulfonyl-isoharnstoffäther, -isothioharnstoffäther, -parabansäuren oder halogenameisensäureamidine spaltet,

c) in

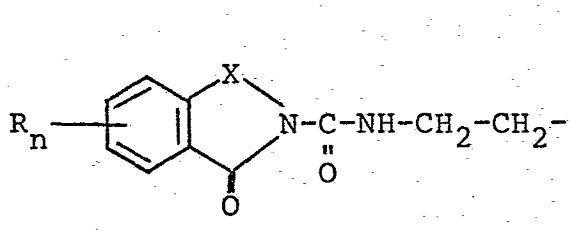

substituierten 1-Piperidinsulfonylthioharnstoffen das Schwefelatom durch Sauerstoff ersetzt,

d) in 1-Piperidinsulfonylharnstoffen der Formel

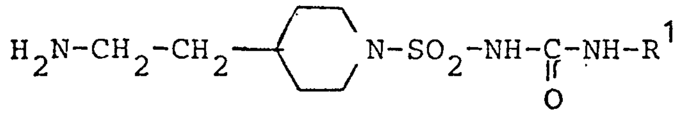

gegebenenfalls stufenweise den Rest

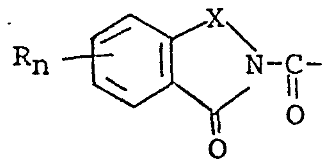

einführt und die Reaktionsprodukte gegebenenfalls zur Salzbildung mit alkalischen Mitteln behandelt.

Die erwähnten 1-Piperidinsulfonyl-carbaminsäureester bzw. -thiolcarbaminsäureester können in der Alkoholkomponete einen Alkylrest oder einen Arylrest oder auch einen heterocyclischen Rest aufweisen. Da dieser Rest bei der Reaktion abgespalten wird, hat seine chemische Konstitution keinen Einfluß auf den Charakter des Endproduktes und kann deshalb in weiten Grenzen variiert werden. Das gleiche gilt für die N-$R^1$-substituierten Carbaminsäureester bzw. die entsprechenden Thiolcarbaminsäureester.

Als Carbaminsäurehalogenide eignen sich in erster Linie die Chloride.

Die als Ausgangsstoffe des Verfahrens infrage kommenden 1-Piperidinsulfonylharnstoffe können an der der Sulfonylgruppe abgewandten Seite des Harnstoffmoleküls unsubstituiert oder ein- oder insbesondere zweifach substituiert sein. Da diese Substituenten bei der Reaktion mit Aminen abgespalten werden, kann ihr Charakter in weiten Grenzen variiert werden. Neben alkyl-, -aryl-, acyl- oder heterocyclisch substituierten 1-Piperidinsulfonylharnstoffen kann man auch 1-Piperidinsulfonylcarbamoylimidazole und ähnliche Verbindungen oder Bispiperidinsulfonylharnstoffe, die an einem der Stickstoffatome noch einen weiteren Substituenten z.B. Methyl, tragen können, verwenden. Man

kann beispielsweise derartige Bis-(piperidinsulfonyl)-harnstoffe oder auch N-Piperidinsulfonyl-N'-acylharnstoffe mit $R^1$-substituierten Aminen behandeln und die erhaltenen Salze auf erhöhte Temperaturen, insbesondere solche oberhalb 100°C erhitzen.

Weiterhin ist es möglich, von $R^1$-substituierten Harnstoffen auszugehen oder von solchen $R^1$-substituierten Harnstoffen, die am freien Stickstoffatom noch ein- oder insbesondere zweifach substituiert sind und diese mit

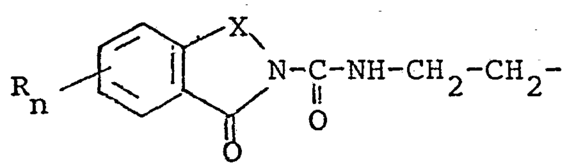

in 4-Stellung substituierten 1-Piperidinsulfonamiden umzusetzen. Als solche Ausgangsstoffe kommen beispielsweise infrage N-Cyclohexyl-harnstoff, die entsprechenden N'-Acetyl, N'-Nitro, N'-Cyclohexyl, N',N'-Diphenyl-(wobei die beiden Phenylreste auch substituiert sowie direkt oder auch über ein Brückenglied wie $-CH_2-$, -NH-, -O- oder S- miteinander verbunden sein können) N'-Methyl-N'-phenyl-, N',N'-Dicyclohexylharnstoffe sowie Cyclohexyl-carbamoyl-imidazole, -pyrazole oder -triazole sowie solche der genannten Verbindungen, die anstelle des Cyclohexyls einen anderen im Bereich der Definition für $R^1$ liegenden Substituenten tragen.

Die Spaltung der als Ausgangsstoffe genannten 1-Piperidinsulfonylparabansäuren, -isoharnstoffäther, -isothioharnstoffäther oder -halogenameisensäureamidine erfolgt zweckmäßig durch alkalische Hydrolyse. Isoharnstoffäther können auch in einem sauren Medium mit gutem Erfolg gespalten werden.

Der Ersatz des Schwefelatoms in der Thioharnstoffgruppierung von entsprechend substituierten 1-Piperidinsulfonylthioharnstoffen durch ein Sauerstoffatom kann in bekannter

Weise zum Beispiel mit Hilfe von Oxyden oder Salzen von Schwermetallen oder auch durch Anwendung von Oxydationsmitteln, wie Wasserstoffperoxid, Natriumperoxid, salpetriger Säure oder Permanganaten ausgeführt werden. Die Thioharnstoffe können auch entschwefelt werden durch Behandlung mit Phosgen oder Phosphorpentachlorid. Als Zwischenstufe erhaltene Chlorameisensäureamidine bzw. Carbodiimide können durch geeignete Maßnahmen wie Verseifen oder Anlagern von Wasser in die 1-Piperidinsulfonylharnstoffe überführt werden.

Die Acylierung der Sulfonylharnstoffe gemäß Verfahren d) kann mit reaktiven Derivaten der Säure

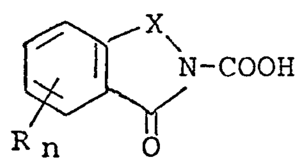

wie beispielsweise Halogeniden oder Urethanen erfolgen.

Die Herstellung der physiologisch verträglichen Salze erfolgt nach an sich bekannten Methoden. Zur Salzbildung sind insbesondere geeignet Alkali- und Erdalkalihydroxyde, -carbonate oder -bicarbonate sowie physiologisch verträgliche organische Basen.

Die Ausführungsformen des Verfahrens gemäß der Erfindung können im allgemeinen hinsichtlich der Reaktionsbedingungen weitgehend variiert und den jeweiligen Verhältnissen angepaßt werden. Beispielsweise können die Umsetzungen in Abwesenheit oder Anwesenheit von Lösungsmitteln, bei Raumtemperatur oder bei erhöhter Temperatur durchgeführt werden.

Je nach dem Charakter der Ausgangsstoffe kann das eine oder andere der beschriebenen Verfahren in einzelnen Fällen einen gewünschten individuellen 1-Piperidinsulfonylharnstoff nur in geringen Ausbeuten liefern oder zu dessen Synthese nicht geeignet sein. In solchen verhältnismäßig

selten auftretenden Fällen macht es dem Fachmann keine Schwierigkeiten, das gewünschte Produkt auf einem anderen der beschriebenen Verfahrenswege zu synthetisieren.

Die erhaltenen Verbindungen können durch Umfällen und/oder Umkristallisieren gereinigt werden. Die Reinigung kann auch erfolgen, indem man die Substanz aus einem kristallinen (Alkali-)Salz in einem geeigneten Lösungsmittel in Freiheit setzt.

Die erfindungsgemäßen Verbindungen zeichnen sich durch wertvolle pharmakologische Eigenschaften, insbesondere blutzuckersenkende, aus. Sie eignen sich daher als Arzneimittel, insbesondere als Antidiabetika.

Die blutzuckersenkende Wirkung der beschriebenen Piperidinsulfonylharnstoffe kann dadurch festgestellt werden, daß man sie als freie Verbindungen oder in Form der Natriumsalze in Dosen von 10 mg oder 2 mg/kg an normal ernährte Kaninchen verfüttert und den Blutzuckerwert nach der bekannten Methode von Hagedorn-Jensen oder mit einem Autoanalyzer über eine längere Zeitdauer ermittelt.

Die Bestimmung der blutzuckersenkenden Wirkung kann aber auch mit geringen Dosen oder nach anderen bekannten Methoden erfolgen.

N-Cyclohexyl-N'-(4-<2-(1-oxo-isoindolin-2-yl-carboxamido)-äthyl>-piperidinsulfonyl)-harnstoff wurde in einer Dosierung von 2 mg/kg Kaninchen oral verabreicht und die Blutzuckersenkung wurde mit einem Autoanalyzer über eine längere Zeitdauer ermittelt. Die Blutzuckersenkung betrug nach 1, 3, 6, 24 bzw. 48 Stunden 24, 34, 35, 31 bzw. 20 %. Selbst 72 Stunden nach Verabreichung wurde noch eine Senkung von 10 % beobachtet.

Die erfindungsgemäßen Acylureidoalkylpiperidinsulfonylharnstoffe zeichnen sich durch eine starke und langanhaltende blutzuckersenkende Wirkung aus.

Die Eigenschaften der Verbindungen erlauben es, in der Therapie des Diabetes mellitus mit so geringen Dosen auszukommen, daß das Präparat nur die verminderte Ansprechbarkeit des Pankreas auf einen erhöhten Blutzuckerspiegel wieder normalisiert.
Benzolsulfonylharnstoffe mit Ureidoalkylrest sind schon mehrfach beschrieben worden (DE-PS 14 43 911, DE-AS 16 70 700, DE-PS 16 18 389, DE-PS 22 38 870). 1-Piperidinsulfonylharnstoffe mit Acylureidoalkylrest sind noch nicht bekannt und es war nicht zu erwarten, daß sie sich durch die oben erwähnten günstigen Eigenschaften auszeichnen.

Die beschriebenen Sulfonylharnstoffe sollen vorzugsweise zur Herstellung von oral verabreichbaren Präparaten zur Behandlung des Diabetes mellitus dienen. Sie können als solche oder in Form ihrer Salze bzw. in Gegenwart von Stoffen, die zu einer Salzbildung führen, appliziert werden. Zur Salzbildung können beispielsweise alkalische Mittel wie Alkali- oder Erdalkalihydroxyde, -carbonate oder -bicarbonate herangezogen werden. Die Präparate können neben dem Sulfonylharnstoff bzw. dessen Salz auch noch andere Wirkstoffe enthalten.
Als medizinische Präparate kommen vorzugsweise Tabletten in Betracht, die neben den Verfahrenserzeugnissen die üblichen Träger- und Hilfsmittel wie Talkum, Stärke, Milchzucker oder Magnesiumstearat enthalten.
Ein Präparat, das die erfindungsgemäßen 1-Piperidinsulfonylharnstoffe als Wirkstoff enthält, z.B. eine Tablette oder ein Pulver mit oder ohne Zusätze, ist zweckmäßig in eine geeignet dosierte Form gebracht. Als Dosis ist dabei eine solche zu wählen, die der Wirksamkeit des verwendeten 1-Piperidinsulfonylharnstoffs und dem gewünschten Effekt angepaßt ist. Zweckmäßig beträgt die Dosierung je Einheit etwa 0,1 bis 10 mg, vorzugsweise 0,5 bis 2 mg, jedoch können auch darüber oder darunter liegende Dosierungseinheiten verwendet werden, die gegebenenfalls vor Applikation zu teilen bzw. zu vervielfachen sind.

Die nachfolgenden Beispiele zeigen einige der zahlreichen Verfahrensvarianten, die zur Synthese der erfindungsgemäßen Sulfonylharnstoffe verwendet werden können. Sie sollen jedoch nicht eine Einschränkung des Erfindungsgegenstandes darstellen.

Beispiel 1:

N-Cyclohexyl-N'-(4-⟨2-(1-oxo-isoindolin-2-yl-carboxamido)-äthyl⟩-piperidinsulfonyl)-harnstoff

2,7 g 4-(2-⟨1-Oxo-isoindolin-2-yl-carboxamido⟩-äthyl)-1-piperidinsulfonamid werden in 50 ml Aceton und 25 ml Dioxan nach Zusatz von 2,1 g gemahlener Pottasche 3 Std. unter Rückfluß gerührt. Nach kurzem Abkühlen tropft man 1,05 g Cyclohexylisocyanat, gelöst in wenig Aceton, zu und rührt weitere 5 Stunden unter Rückfluß. Nach dem Erkalten verdünnt man mit Wasser und säuert die Lösung mit 2N Salzsäure an. Der Niederschlag wird abgesaugt und aus Äthanol umkristallisiert. Der so hergestellte N-Cyclohexyl-N'-(4-⟨2-(1-oxo-isoindolin-2-yl-carboxamido)-äthyl⟩-piperidinsulfonyl)-harnstoff schmilzt bei 176 - 177°C.

Das als Ausgangsmaterial verwendete Sulfonamid wird auf folgendem Weg erhalten:
1-Oxo-isoindolin-2-yl-carbonsäurechlorid (hergestellt aus 1-Oxo-isoindolin-Na mit Phosgen) wird mit 4-(2-Amino-äthyl)-pyridin zum 4-(2-⟨1-Oxo-isoindolin-2-yl-carboxamido⟩-äthyl)-pyridin vom Schmp. 158 - 160°C umgesetzt. Durch Hydrierung mit Platinoxid als Katalysator wird das 4-(2-⟨1-Oxo-isoindolin-2-yl-carboxamido⟩-äthyl)-piperidin vom Schmp. 138 - 140°C erhalten. Die Reaktion dieses Produkts mit Sulfamid liefert das oben eingesetzte 4-(2-⟨1-Oxo-isoindolin-2-yl-carboxamido⟩-äthyl)-1-piperidinsulfonamid vom Schmp. 210 - 212°C.

In analoger Weise erhält man den

N-Butyl-N'-(4-⟨2-(1-oxo-isoindolin-2-yl-carboxamido)-äthyl⟩-piperidinsulfonyl)-harnstoff vom Schmp. 154 - 155°C (aus Äthanol)

N-(4-Methyl-cyclohexyl)-N'-(4-⟨2-(1-oxo-isoindolin-2-yl-carboxamido)-äthyl⟩-piperidinsulfonyl)-harnstoff vom Schmp. 199 - 200°C (aus Äthanol)

N-(4-Äthyl-cyclohexyl)-N'-(4-⟨2-(1-oxo-isoindolin-2-yl-carboxamido)-äthyl⟩-piperidinsulfonyl)-harnstoff vom Schmp. 186 - 187°C (aus Äthanol)

N-($\Delta^3$-Cyclohexenyl)-N'-(4-⟨2-(1-oxo-isoindolin-2-yl-carboxamido)-äthyl⟩-piperidinsulfonyl)-harnstoff vom Schmp. 190 - 191°C (aus Äthanol)

N-Bicyclo/2.2.1/hept-2-yl-methyl-N'-(4-⟨2-(1-oxo-isoindolin-2-yl-carboxamido)-äthyl⟩-piperidinsulfonyl)-harnstoff vom Schmp. 167 - 169°C (aus Äthanol)

N-Propyl-N'-(4-⟨2-(1-oxo-isoindolin-2-yl-carboxamido)-äthyl⟩-piperidinsulfonyl)-harnstoff vom Schmp. 186 - 187°C (aus Äthanol)

N-Benzyl-N'-(4-⟨2-(1-oxo-isoindolin-2-yl-carboxamido)-äthyl⟩-piperidinsulfonyl)-harnstoff vom Schmp. 183 - 184°C (aus Äthanol-Dimethylformamid)

N-Cyclopentyl-N'-(4-⟨2-(1-oxo-isoindolin-2-yl-carboxamido)-äthyl⟩-piperidinsulfonyl)-harnstoff vom Schmp. 172 - 173°C (aus Äthanol)

N-Cycloheptyl-N'-(4-⟨2-(1-oxo-isoindolin-2-yl-carboxamido)-äthyl⟩-piperidinsulfonyl)-harnstoff vom Schmp. 154 - 155°C (aus Äthanol)

N-Cyclooctyl-N'-(4-⟨2-(1-oxo-isoindolin-2-yl-carboxamido)-äthyl⟩-piperidinsulfonyl)-harnstoff vom Schmp. 151 - 152°C (aus Äthanol)

N-Hexyl-N'-(4-⟨2-(1-oxo-isoindolin-2-yl-carboxamido)-äthyl⟩-piperidinsulfonyl)-harnstoff vom Schmp. 133 - 134°C (aus Äthanol)

N-Cyclopentylmethyl-N'-(4-⟨2-(1-oxo-isoindolin-2-yl-carboxamido)-äthyl⟩-piperidinsulfonyl)-harnstoff vom Schmp. 169 - 170°C (aus Äthanol)

N-Cyclobutyl-N'-(4-⟨2-(1-oxo-isoindolin-2-yl-carboxamdio)-äthyl⟩-piperidinsulfonyl)-harnstoff vom Schmp. 175 - 177°C (aus Äthanol)

N-(4,4-Dimethyl-cyclohexyl)-N'-(4-⟨2-(1-oxo-isoindolin-2-yl-carboxamido)-äthyl⟩-piperidinsulfonyl)-harnstoff vom Schmp. 171 - 173°C (aus Äthanol)

N-(3-Methyl-cyclopentylmethyl)-N'-(4-⟨2-(1-oxo-isoindolin-2-yl-carboxamido)-äthyl⟩-piperidinsulfonyl)-harnstoff vom Schmp. 167 - 168°C (aus Äthanol)

Beispiel 2:

N-(4-Methoxy-cyclohexyl)-N'-(4-⟨2-(1-oxo-isoindolin-2-yl-carboxamido)-äthyl⟩-piperidinsulfonyl)-harnstoff

2,12 g N-(4-⟨2-(1-Oxo-isoindolin-2-yl-carboxamido)-äthyl⟩-1-piperidinsulfonyl)-carbamidsäuremethylester (Schmp. 170 - 172°C, hergestellt durch Umsetzung von 4-(2-⟨1-Oxo-isoindolin-2-yl-carboxamido⟩-äthyl)-1-piperidinsulfonamid mit Chlorameisensäuremethylester) werden mit 1,29 g 4-Methoxy-cyclohexylamin in 50 ml Dioxan 3 Stunden unter Rückfluß gerührt. Nach dem Erkalten engt man im Vakuum ein, nimmt den Rückstand in verdünnter Ammoniaklösung auf, filtriert und säuert mit 2 N Salzsäure an. Das zunächst ölig anfallende Produkt wird mit Chloroform extrahiert und die Chloroform-Lösung nach dem Trocknen über Natriumsulfat eingedampft. Der Rückstand wird aus Essigester umkristallisiert. Der so erhaltene N-(4-Methoxy-cyclohexyl)-N'-(4-⟨2-(1-oxo-isoindolin-2-yl-carboxamido)-äthyl⟩-piperidinsulfonyl)-harnstoff schmilzt bei 163 - 164°C.

N-Bicyclo/2.2.1/hept-2-yl-N'-(4-⟨2-(1-oxo-isoindolin-2-yl-carboxamido)-äthyl⟩-piperidinsulfonyl)-harnstoff vom Schmp. 145 - 147°C (aus Äthanol-Dimethylformamid)

N-($\Delta^3$-Cyclohexenyl-methyl)-N'-(4-⟨2-(1-oxo-isoindolin-2-yl-carboxamdio)-äthyl⟩-piperidinsulfonyl)-harnstoff vom Schmp. 193 - 195°C (aus Äthanol-Dimethylformamid)

N-(Bicyclo⟨2,2,1⟩-hept-5-en-2-yl-methyl)-N'-(4-⟨2-(1-oxo-isoindolin-2-yl-carboxamido)-äthyl⟩-piperidinsulfonyl)-harnstoff
vom Schmp. 178 - 180°C (aus Äthanol)

N-(4-Chlor-cyclohexyl)-N'-(4-<2-(1-oxo-isoindolin-2-yl-carboxamido)-äthyl>-piperidinsulfonyl)-harnstoff
vom Schmp. 199 - 201°C (aus Essigester)

Beispiel 3:

N-Cyclohexyl-N'-(4-⟨2-(1-oxo-isoindolin-2-yl-carboxamido)-äthyl⟩-piperidinsulfonyl)-harnstoff

0,51 g N-Cyclohexyl-N'-(4-⟨2-(1-oxo-isoindolin-2-yl-carboxamido)-äthyl⟩-piperidinsulfonyl)-thioharnstoff (Schmp. 165 - 166°C, hergestellt aus dem entsprechenden Sulfonamid mit Cyclohexylsenföl) werden in 25 ml Wasser und 25 ml Methanol mit 0,21 g gelbem Quecksilberoxid vier Stunden auf 55 - 60°C (Badtemperatur) erwärmt. Nach dem Erkalten saugt man das Quecksilbersulfid ab, engt das Filtrat ein und kristallisiert aus Äthanol um. Der so dargestellte N-Cyclohexyl-N'-(4-⟨2-(1-oxo-isoindolin-2-yl-carboxamido)-äthyl⟩-piperidinsulfonyl)-harnstoff schmilzt bei 175 - 177°C.

Beispiel 4:

**N-Cyclohexyl-N'-(4-⟨2-(1-oxo-isoindolin-2-yl-carboxamido)-äthyl⟩-piperidinsulfonyl)-harnstoff**

0,76 g N-Cyclohexyl-N'-(4-⟨2-(1-oxo-isoindolin-2-yl-carboxamido)-äthyl⟩-piperidinsulfonyl)-thioharnstoff (Schmp. 165 - 166°C, hergestellt aus dem entsprechenden Sulfonamid mit Cyclohexylsenföl) werden in 30 ml abs. Methanol nach Zugabe von 0,32 g Quecksilberoxid sechs Stunden auf 50 - 55°C (Badtemperatur) erwärmt. Man läßt abkühlen, filtriert und dampft im Vakuum ein. Der als Rückstand erhaltene N-Cyclohexyl-N'-(4-⟨2-(1-oxo-isoindolin-2-yl-carboxamido)-äthyl⟩-piperidinsulfonyl)-isoharnstoffmethyläther schmilzt bei 105 - 107°C.

0,5 g des so hergestellten Isoharnstoffmethyläther werden in 15 ml conc. Salzsäure zwei Stunden bei 50°C gerührt. Nach dem Verdünnen mit 20 ml Wasser saugt man ab und kristallisiert den N-Cyclohexyl-N'-(4-⟨2-(1-oxo-isoindolin-2-yl-carboxamido)-äthyl⟩-piperidinsulfonyl)-harnstoff aus Äthanol um. Er schmilzt bei 174 - 176°C und zeigt mit einer auf andere Weise hergestellten Probe keine Schmelzpunktdepression.

Beispiel 5:

N-Cyclohexyl-N'-(4-<2-(1-oxo-3-methyl-isoindolin-2-yl-carboxamido)-äthyl>-piperidinsulfonyl)-harnstoff

5,88 g 1-Oxo-3-methyl-isoindolin werden in 100 ml abs. Toluol mit 1,92 g 55 - 60 %iger Natriumhydrid-Dispersion 4 Stunden unter Rückfluß gerührt. Die abgekühlte Mischung wird portionsweise zu 70 ml ca. 15 %iger Phosgen-Toluol-Lösung gegossen und weitere 2 Stunden auf 40 - 50° erwärmt. Dann saugt man den anorganischen Niederschlag ab und engt die Lösung ein. 1,2 g des so erhaltenen 1-Oxo-3-methyl-isoindolin-2-carbamoyl-chlorids werden in 50 ml Methylenchlorid gelöst und bei Raumtemperatur zu einer Suspension von 2,4 g N-(4-<2-Aminoäthyl>-piperidinsulfonyl)-N'-cyclohexyl-harnstoff in 50 ml Methylenchlorid und 1,54 g Natriumcarbonat in 15 ml Wasser getropft. Man rührt 3 Stunden bei Raumtemperatur nach und säuert dann mit 2N HCl an. Die organische Phase wird abgetrennt, über $Na_2SO_4$ getrocknet und im Vakuum eingeengt. Der ölige Rückstand wird 1 x aus verdünntem Ammoniak mit verdünnter Salzsüure ausgefällt und zur endgültigen Reinigung über eine Kieselgel-Säule (Laufmittel Chloroform: Methanol = 20 : 1) geschickt. Der auf diese Weise hergestellte N-Cyclohexyl-N'-(4-<1-oxo-3-methyl-isoindolin-2-yl-carboxamido)-äthyl>-piperidinsulfonyl)-harnstoff schmilzt bei 143 - 45°C und wurde aus Äthanol umkristallisiert.
Der als Ausgangsmaterial eingesetzte N-(4-<2-Aminoäthyl>-piperidinsulfonyl)-N'-cyclohexyl-harnstoff wurde auf folgende Weise synthetisiert:

4-(Aminoäthyl)-pyridin werden in Methylenchlorid mit Acetylchlorid versetzt. Dieses dabei erhaltene 4-(2-<Acetylamino>-äthyl)-pyridin wird in Methanol nach Zusatz von äthanolischer Salzsäure mit Platinoxid als Katalysator zum 4-(2-<Acetylamino>-äthyl)-piperidin (ölig) hydriert. Aus diesem gewinnt man durch Reaktion mit Sulfamid in Pyridin unter Rückfluß das 4-(2-<Acetylamino>-äthyl)-piperidin-1-sulfonamid vom Schmelzpunkt 205 - 06°C. Aus dem Sulfonamid erhält man durch Umsetzung mit Kaliumcarbonat und Cyclohexylisocyanat in Butanon-(2) den N-(4-⟨2-(Acetylamino)-äthyl⟩-piperidinsulfonyl)-N'-cyclohexylharnstoff (Schmp. 175 - 176°C (aus Äthanol)), der durch Verseifung mit siedender wäßriger Natronlauge in den N-(4-⟨2-Aminoäthyl⟩-piperidinsulfonyl)-N'-cyclohexylharnstoff vom Schmp. 218 - 220°C überführt wird.

Patentansprüche:

1. Sulfonylharnstoffe der Formel

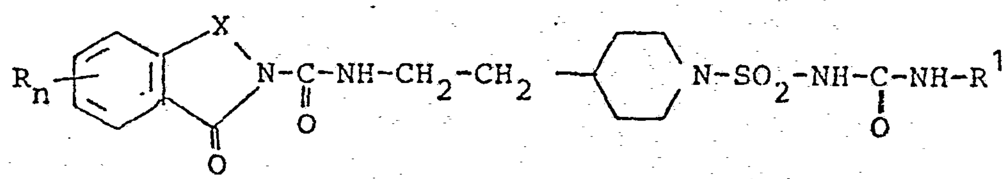

in welcher bedeuten

n 1 oder 2

R Wasserstoff, Alkyl mit 1 bis 4 Kohlenstoffatomen, Alkoxy mit 1 bis 4 Kohlenstoffatomen oder Halogen, wobei falls n 2 ist, die R gleich oder verschieden sein können,

X eine $-CH_2-$, $-CH_2-CH_2-$ oder eine $-CH(CH_3)-$Gruppe

$R^1$ Alkyl von 2 bis 8 C-Atomen, Cycloalkyl, Alkylcycloalkyl, Dialkylcycloalkyl, Cycloalkylalkyl, Cycloalkenyl, Alkylcycloalkenyl mit jeweils 5 bis 9 C-Atomen, Methylcyclopentylmethyl Cyclohexenylmethyl, Chlorcyclohexyl, Methoxycyclohexyl, Bicycloheptyl, Bicycloheptenyl, Bicycloheptylmethyl, Bicycloheptenylmethyl, Bicyclooctyl, Nortricyclyl, Adamantyl oder Benzyl

und deren physiologisch verträglichen Salze.

2. Verfahren zur Herstellung von Sulfonylharnstoffen gemäß Anspruch 1, dadurch gekennzeichnet, daß man

a) mit der Gruppe

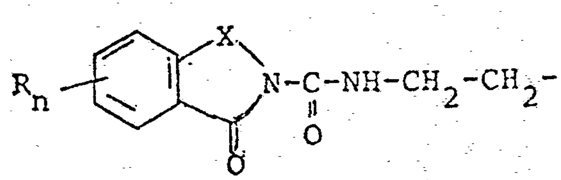

in 4-Stellung substituierte 1-Piperidinsulfonyl-isocyanate, -carbaminsäureester, -thiolcarbaminsäureester, -harnstoffe, -semicarbazide oder -semicarbazone mit einem Amin $R^1$-$NH_2$ oder dessen Salzen umsetzt oder Sulfonamide der Formel

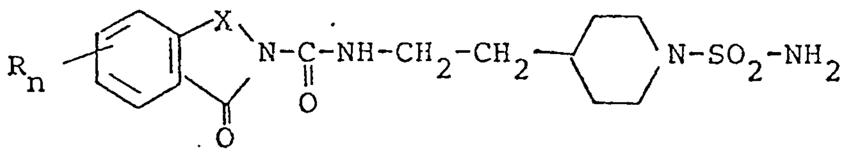

oder deren Salze mit $R^1$-substituierten Isocyanaten, Carbaminsäureestern, Thiolcarbaminsäureestern, Carbaminsäurehalogeniden oder Harnstoffen umsetzt,

b) entsprechend substituierte 1-Piperidinsulfonyl-isoharnstoffäther, -isothioharnstoffäther, -parabansäuren oder halogenameisensäureamidine spaltet,

c) in

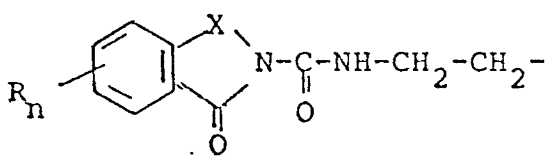

substituierten 1-Piperidinsulfonylthioharnstoffen das Schwefelatom durch Sauerstoff ersetzt,

d) in 1-Piperidinsulfonylharnstoffen der Formel

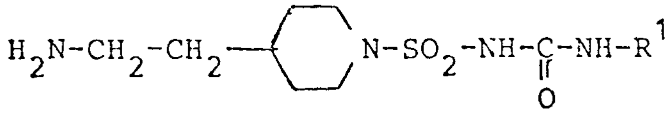

gegebenenfalls stufenweise den Rest

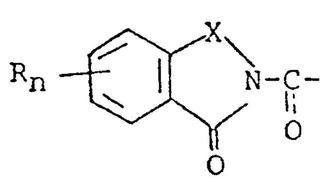

einführt
und die Reaktionsprodukte gegebenenfalls zur Salzbildung mit alkalischen Mitteln behandelt.

3. Arzneimittel auf Basis eines Sulfonylharnstoffs gemäß Anspruch 1 oder eines seiner Salze.

4. Verwendung eines Sulfonylharnstoffs gemäß Anspruch 1 oder eines seiner Salze bei der Bekämpfung von Diabetes.

5. Verfahren zur Herstellung eines Arzneimittels gemäß Anspruch 3, dadurch gekennzeichnet, daß man einen Sulfonylharnstoff der im Anspruch 1 angegebenen Formel oder eines seiner Salze in eine geeignete Applikationsform bringt.

6. Verfahren zur Senkung des Blutzuckerspiegels bei der Behandlung des Diabetes, dadurch gekennzeichnet, daß man eine wirksame Menge einer Verbindung gemäß Anspruch 1 verabreicht.

Patentanspruch für Österreich

Verfahren zur Herstellung von Sulfonylharnstoffen der Formel

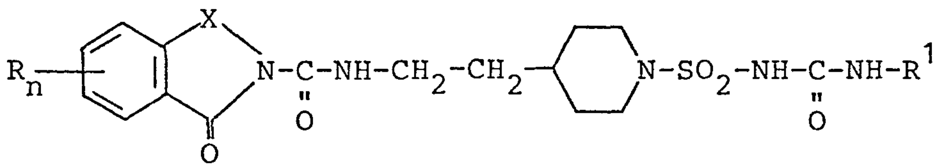

in welcher bedeuten

n 1 oder 2

R Wasserstoff, Alkyl mit 1 bis 4 Kohlenstoffatomen, Alkoxy mit 1 bis 4 Kohlenstoffatomen oder Halogen, wobei falls n 2 ist, die R gleich oder verschieden sein können,

X eine $-CH_2-$, $-CH_2-CH_2-$ oder eine $-CH(CH_3)-$Gruppe

$R^1$ Alkyl von 2 bis 8 C-Atomen, Cycloalkyl, Alkylcycloalkyl, Dialkylcycloalkyl, Cycloalkylalkyl, Cycloalkenyl, Alkylcycloalkenyl mit jeweils 5 bis 9 C-Atomen, Methylcyclopentylmethyl Cyclohexenylmethyl, Chlorcyclohexyl, Methoxycyclohexyl, Bicycloheptyl, Bicycloheptenyl, Bicycloheptylmethyl, Bicycloheptenylmethyl, Bicyclooctyl, Nortricyclyl, Adamantyl oder Benzyl

und von deren physiologisch verträglichen Salzen, dadurch gekennzeichnet, daß man

a) mit der Gruppe

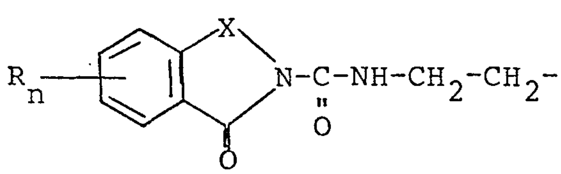

in 4-Stellung substituierte 1-Piperidinsulfonyl-isocyanate, -carbaminsäureester, -thiolcarbaminsäureester, -harnstoffe, -semicarbazide oder -semicarbazone mit einem Amin $R^1-NH_2$ oder dessen Salzen umsetzt oder Sulfonamide der Formel

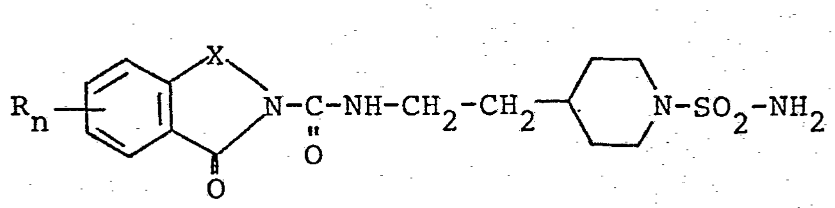

oder deren Salze mit $R^1$-substituierten Isocyanaten, Carbaminsäureestern, Thiolcarbaminsäureestern, Carbaminsäurehalogeniden oder Harnstoffen umsetzt,

b) entsprechend substituierte 1-Piperidinsulfonyl-isoharnstoffäther, -isothioharnstoffäther, -parabansäuren oder halogenameisensäureamidine spaltet,

c) in

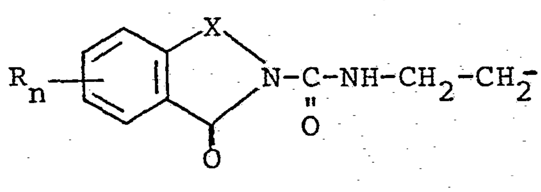

substituierten 1-Piperidinsulfonylthioharnstoffen das Schwefelatom durch Sauerstoff ersetzt,

d) in 1-Piperidinsulfonylharnstoffen der Formel

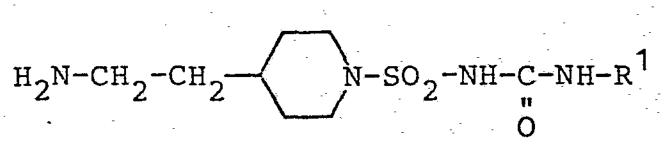

gegebenenfalls stufenweise den Rest

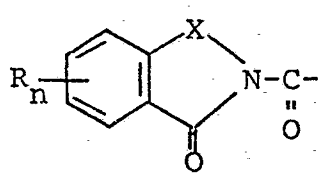

einführt

und die Reaktionsprodukte gegebenenfalls zur Salzbildung mit alkalischen Mitteln behandelt.

0030650

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

Nummer der Anmeldung
EP 80107305.7

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | DE - A - 2 355 262 (PFIZER)<br>+ Ansprüche 1,8,9,14,18,19; Seiten 8-11 +<br>-- | 1-3,5 | C 07 D 401/12<br>A 61 K 31/64//<br>(C 07 D 401/12<br>C 07 D 209/46<br>C 07 D 211/96)<br>(C 07 D 401/12<br>C 07 D 217/24<br>C 07 D 211/96) |
| | DE - A1 - 2 621 958 (HOECHST)<br>+ Seite 10, 3. Absatz - Seite 14, erster Absatz; Ansprüche+<br>---- | 1-3,5 | |
| | | | RECHERCHIERTE SACHGEBIETE (Int. Cl.³) |
| | | | C 07 D 401/00<br>C 07 D 211/00<br>C 07 D 209/00<br>C 07 D 217/00<br>C 07 D 213/00<br>A 61 K 31/00 |

**UNVOLLSTÄNDIGE RECHERCHE**

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-3,5
Unvollständig recherchierte Patentansprüche: -
Nicht recherchierte Patentansprüche: 4,6
Grund für die Beschränkung der Recherche:

Verfahren zur therapeutischen Behandlung des menschlichen oder tierischen Körpers, Art. 52(4) EPÜ

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| WIEN | 11-02-1981 | TENGLER |

EPA Form 1505.1 06.78